# EUROPEAN PATENT APPLICATION

(11) **EP 2 062 910 A1**
(43) Date of publication of application: **27.05.2009**
(21) Application number: 07121520.6
(22) Date of filing: 26.11.2007
(51) Int. Cl.: C07K 1/14

(54) **Selective enrichment of post-translationally modified proteins and/or peptides from complex samples**

(71) Applicant: Koninklijke Philips Electronics N.V., 5621 BA Eindhoven (NL)
(72) Inventor: The designation of the inventor has not yet been filed
(74) Representative: Plünnecke, Ingo

(57) **Abstract**

The present invention relates to the selective enrichment of post-translationally modified proteins and/or peptides from complex samples by combining a particular protein/peptide labeling and fractionation strategy with specific chemical and/or enzymatic reactions targeting the post-translational modification to be analyzed. In particular, the invention relates to methods for the separation of phospho-proteins and/or - peptides and/or for the discrimination between different subsets of phospho-proteins and/or -peptides.

## Description

### SUBJECT OF THE INVENTION

The present invention relates to the selective enrichment of post-translationally modified proteins and/or peptides from complex samples by combining a particular protein/peptide labeling and fractionation strategy with specific chemical and/or enzymatic reactions targeting the post-translational modification to be analyzed. In particular, the invention relates to methods for the separation of phospho-proteins and/or -peptides and/or for the discrimination between different subsets of phospho-proteins and/or -peptides.

### BACKGROUND OF THE INVENTION

The identification, separation, and analysis of particular proteins or subsets of proteins from complex samples are invaluable for unraveling how biological processes occur at a molecular level or to which degree proteins differ among various cell types or between physiological states.

A major challenge in modem biology is directed to the understanding of the expression, function, and regulation of the entire set of proteins encoded by an organism, a technical field commonly known as proteomics. However, as there is no possibility to amplify proteins, research in this field is generally rather tedious because even a cell extract of a relatively simple prokaryotic organism contains a multitude of proteins encompassing a huge range of concentrations. Therefore, such a task is beyond the capabilities of any current single analytical methods.

Thus, due to the methodological constraints proteome analysis relies not only to methods for identifying and quantifying proteins but - to a considerable extent - also on methods allowing their accurate and reliable separation according to their structural and/or functional properties, with these subsets being then better accessible to further analysis.

The proteome is of dynamic nature, with alterations in protein synthesis, activation, and/or post-translational modification in response to external stimuli or alterations in the cellular environment. Therefore, the proteome's inherent complexity exceeds that of the genome or the transcriptome the mRNA complement of a cell.

Due to the extraordinary amount of data to be processed in such proteomic studies the protein/peptide identification process demands tremendous resolving power. Two methods commonly used to resolve such highly complex mixtures are two-dimensional gel electrophoresis (2D-GE; cf., e.g., O'Farrell, P.H. (1975) J. Biol. Chem. 250, 4007-4021) and (two-dimensional) liquid chromatography ((2D)-LC; cf., e.g., Lipton, M.S. et al. (2002) Proc. Natl. Acad. Sci. USA 99, 11049-11054). The peptides and proteins isolated by 2D-GE or 2D-LC are usually identified by mass spectrometry or by determining amino acid composition and/or amino acid sequence.

However, although useful for many applications these identification techniques have major drawbacks with regard to proteomic studies, where highly complex samples are to be investigated. For example, hydrophobic membrane proteins, highly basic or acidic proteins, very large or very small proteins are often poorly resolved via 2D-GE. Furthermore, the detection (sensitivity) limits of these methods as well as shortcomings in labeling technology do not allow for the reliable analysis of multiple samples in parallel, e.g., for comparing relative protein levels between different disease groups, different progression stages of a disease, and between disease stages vs. healthy controls or for performing high-throughput screening analyses.

Therefore, it is highly desirable to develop methodologies which overcome the above limitations and enable processing of multiple complex samples in parallel.

Another important facet of protein analysis in general and proteomics in particular relates to the possibility to study post-translational protein modifications, which can affect activity and binding of a protein and alter its role within the cell (cf., e.g., Pandey, A. and Mann, M. (2000) Nature 405, 837-846). For example, the (reversible) phosphorylation of proteins is crucial for the regulation of many signal transduction cascades such as G-protein-coupled receptor signaling or phospho-tyrosine kinase signaling, whereas an ubiquitination *inter alia* labels proteins for degradation.

One of the unique features of proteomics is that post-translational modifications can be investigated at a more global level, thus allowing the analysis of the entire subset of proteins comprising a particular modification. The expressed products of a single gene represent a protein population that may contain large amounts of micro-heterogeneity, each different state (i.e. analogous proteins differing in the number of post-translationally modified amino acid residues) adding a large amount of diversity to the expression profile of that protein.

Currently, there are several techniques available, for example mass spectrometry, which can in principle distinguish between analogous proteins or peptides due to the presence or absence of a specific modification. However, these changes are frequently not observed in global proteomic studies due to a limited sensitivity of detection. Thus, in order to study a specific post-translational modification, it would be helpful to enrich a sample for that modification, usually by some form of affinity purification, and/or to separate the enriched subset of modified proteins from that sample. However, the available methods are generally hampered by the requirement to label the proteins with appropriate affinity tags or the need to use specific antibodies or other reagents which might interfere with further analyses. Therefore, such methods based on affinity purification are particularly not suitable for processing multiple samples in parallel.

In addition, it is generally cumbersome to distinguish different subsets of proteins and/or peptides bearing a particular modification (e.g., tyrosine-phosphorylated and serine/threonine-phosphorylated proteins) based on affinity purification protocols.

The study of protein phosphorylation has grown exponentially in recent years as researchers from various disciplines have come to realize that key cellular functions are regulated by the reversible phosphorylation and dephosphorylation of proteins on serine, threonine, and tyrosine residues. At present, only a subset of the known eukaryotic protein kinases and protein phosphatases has been characterized with respect to biological function and protein substrate specificity. To understand more about protein phosphorylation and dephosphorylation, it is necessary to identify the specific amino acid residues that become phosphorylated, because identification of these sites in proteins may reveal which protein kinase regulates the protein and thereby help elucidate the biological function and significance of novel phospho-proteins.

Therefore, it is a continuing need for methods allowing the selective enrichment ofpost-translationally modified proteins and/or peptides from complex samples. In particular, it would be desirable to provide methods for the separation and/or discrimination of phospho-proteins and/or -peptides not only with high sensitivity but also without the requirement of specific reagents. Furthermore, it would also be desirable to provide methods that allow for performing multiplexed analyses.

### OBJECT AND SUMMARY OF THE INVENTION

It is an objective of the present invention to provide novel approaches for the selective enrichment of post-translationally modified peptides and/or proteins, in particular of phospho-proteins and/or -peptides, from complex samples. More specifically, it is an object of the present invention to provide methods for performing multiple such analyses in parallel.

It is a further objective of the present invention to provide methods that allow separating post-translationally modified proteins and/or peptides from their unmodified counterparts and/or to discriminate between different subsets of these modified proteins and/or peptides.

These objectives as well as others which will become apparent from the ensuing description are attained by the subject matter of the independent claims. Some of the preferred embodiments of the present invention are defined by the subject matter of the dependent claims.

In one embodiment, the present invention relates to a method for the selective enrichment and/or separation of phospho-proteins and/or -peptides in a sample, comprising:
(a) single or double chemical labeling of the proteins and/or peptides comprised in the sample
(b) fractionating the proteins and/or peptides;
(c) removing or altering the phosphate-group from at least a first subset of phospho-proteins and/or -peptides;
(d) re-fractionating the proteins and/or peptides;
(e) comparing the fractionation patterns obtained in steps (b) and (d); and
(f) separating the at least first subset of phospho-proteins and/or -peptides modified in step (c) based on the results obtained in step (e). In a preferred embodiment, the fractionation/re-fractionation is performed via isoelectric focusing.

In another preferred embodiment, the method further comprises cleaving the proteins into peptides prior to subjecting them to fractionation.

In another preferred embodiment, the method further comprises single or double chemical labeling of the proteins and/or peptides prior to subjecting them to fractionation. Particularly preferably, the double labeling comprises an isotopic and an isobaric labeling. In specific embodiments, the isotopic labeling is performed prior to cleaving the proteins and/or peptides.

In one preferred embodiment of the inventive method, the at least first subset of phospho-proteins and/or -peptides comprises serine- and threonine-phosphorylated phospho-proteins and/or -peptides. Typically, the phosphate-group is removed chemically via β-elimination.

In another embodiment, after performing step (e) the remaining subset of proteins and/or peptides is subjected to another cycle of steps (a) to (e), and wherein step (b) comprises removing or altering a specific post-translational modification from at least a second subset of phospho-proteins and/or -peptides.

In a further preferred embodiment of the inventive method, the at least second subset of phospho-proteins/peptides comprises tyrosine-phosphorylated phospho-proteins and/or -peptides. Typically, the phosphate-group is removed enzymatically via phosphatases.

In another embodiment, the method further comprises analyzing the separated phospho-proteins and/or -peptides by means of mass spectrometry. In some embodiments, the method is performed in a high-throughput format.

The method of the present invention may be used for performing qualitative and/or quantitative proteomic analyses.

Other embodiments of the present invention will become apparent from the detailed description hereinafter.

### FIGURE LEGENDS

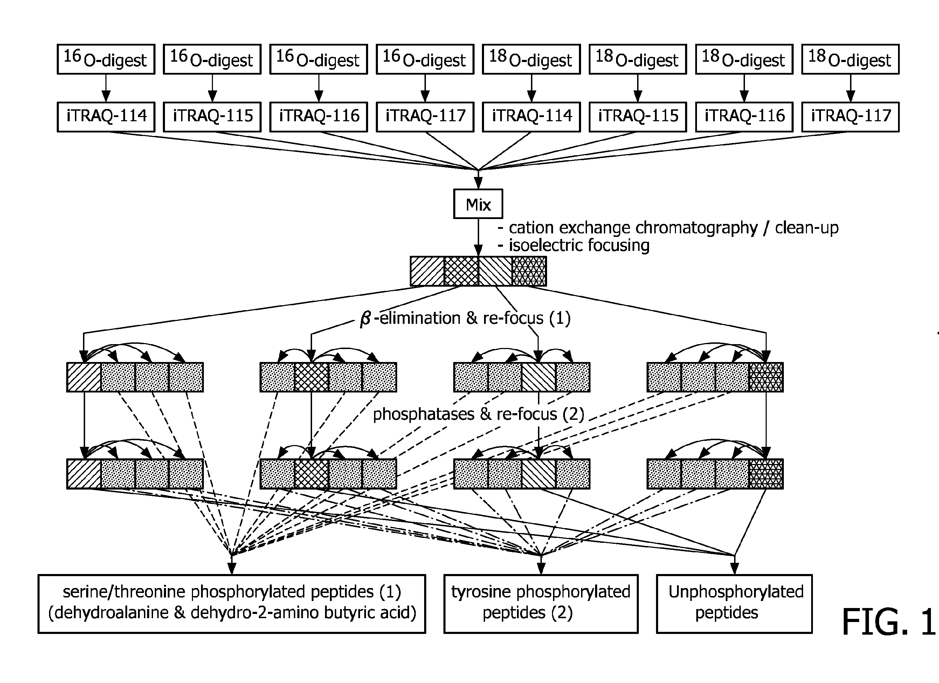

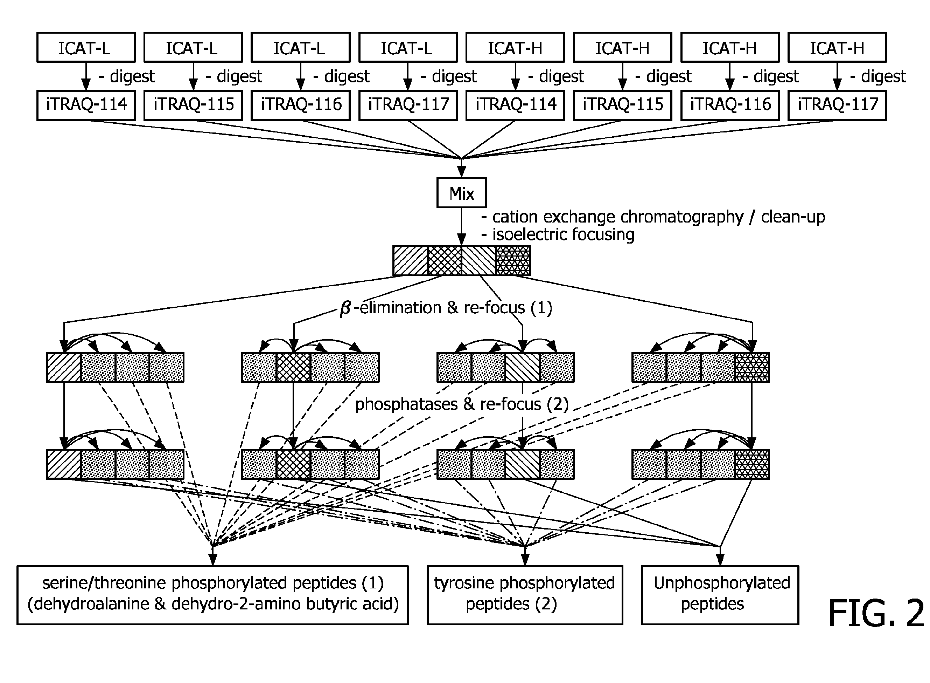

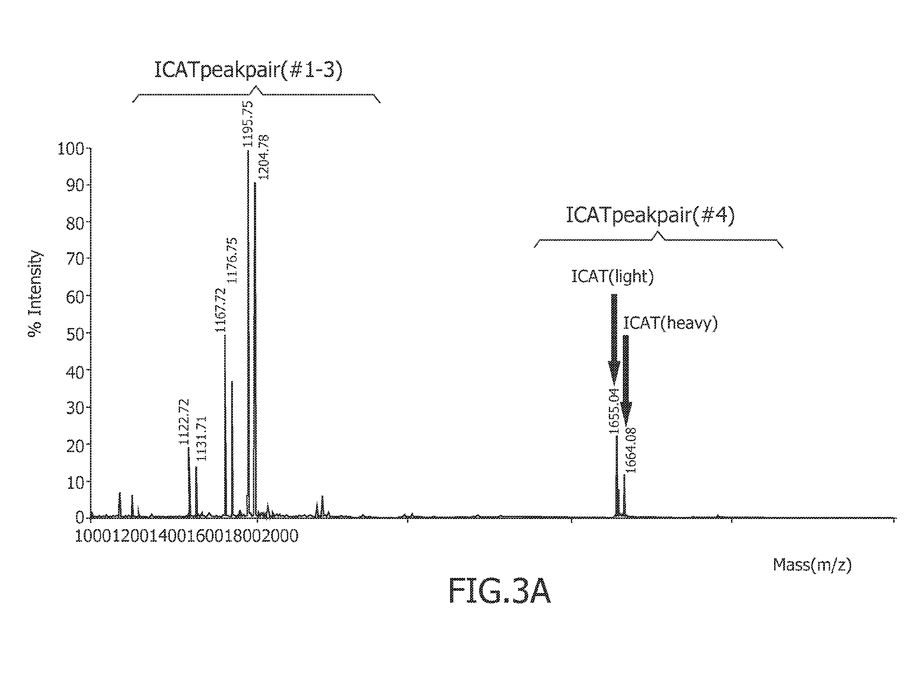

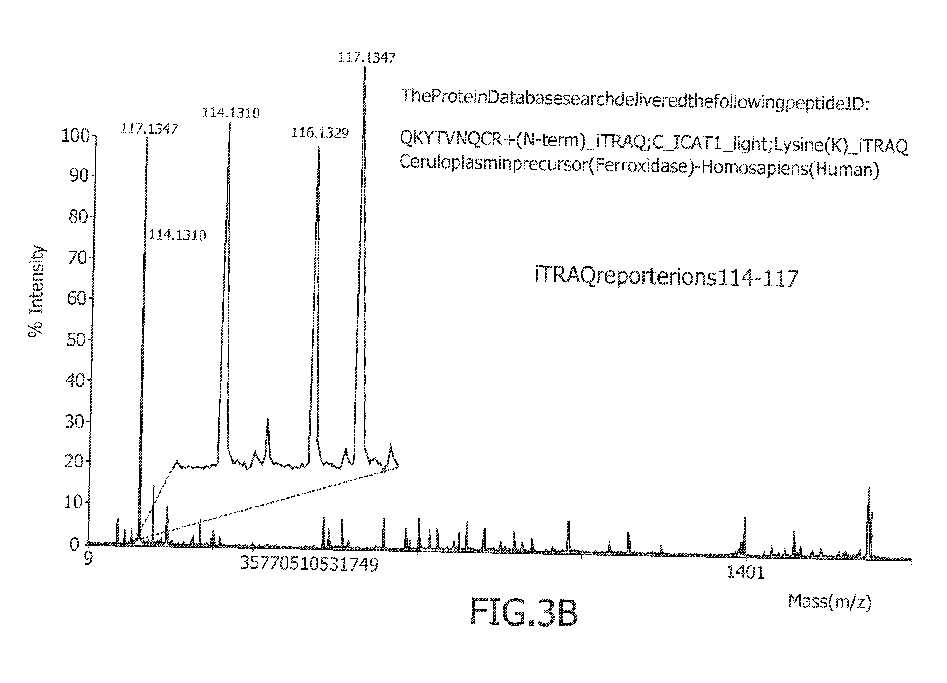

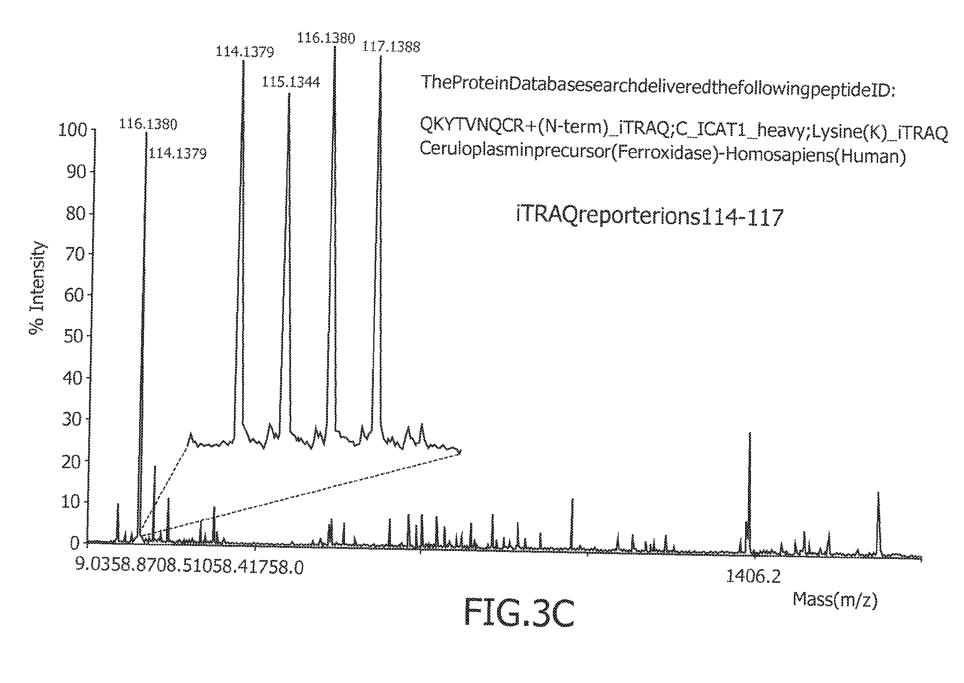

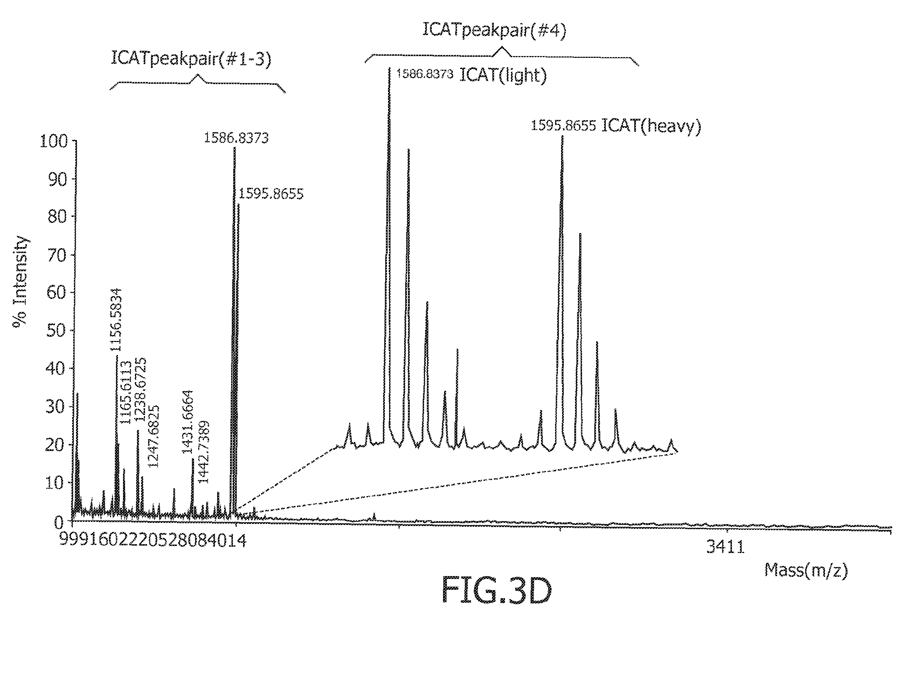

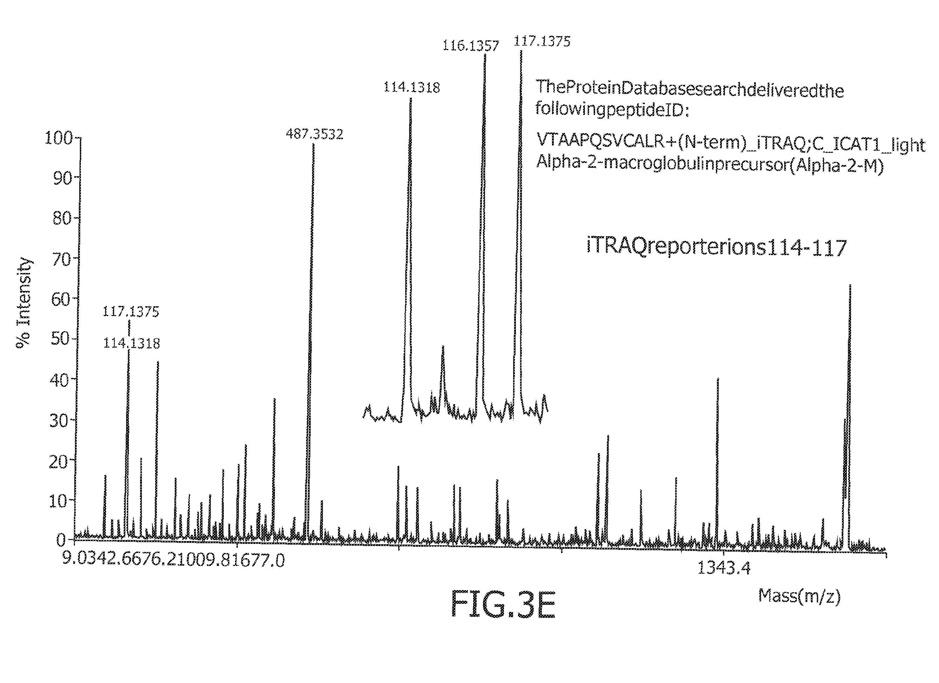

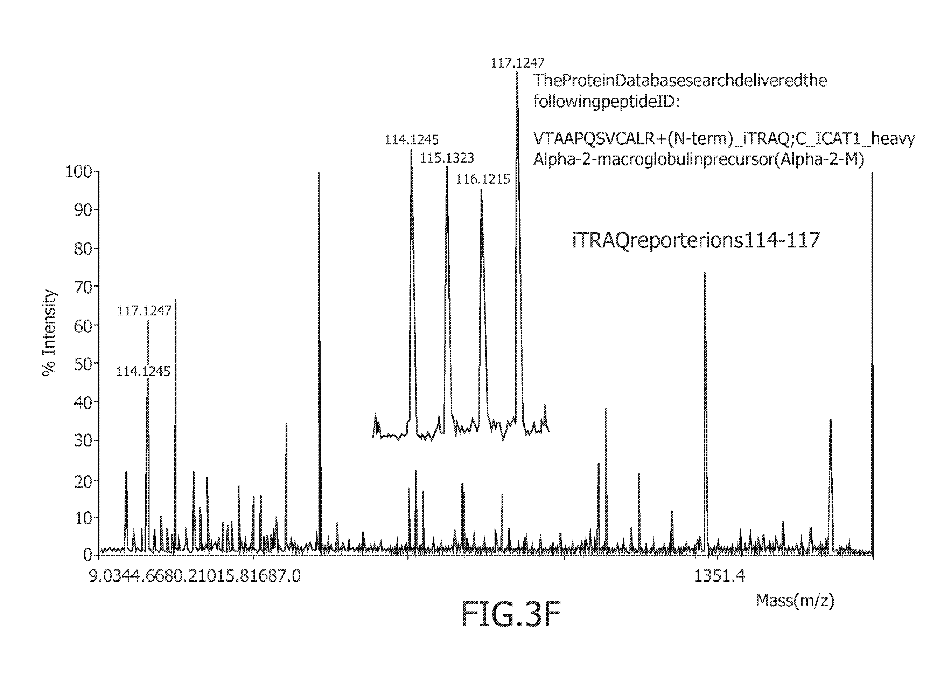

Fig. 1 depicts a schematic illustration of the application of a preferred embodiment of the invention to the selective enrichment of phospho-peptides. The proteins comprised in a given sample are digested in the presence of either ¹⁶O- or ¹⁸O-labeled water (isotopic labeling). Subsequently, the resulting peptides are labeled using the Isobaric Tag for Relative and Absolute Quantitation technology (iTRAQ; isobaric labeling). The labeled peptides are fractionated via isoelectric focusing (IEF). All individual fractions are collected and individually treated with a reaction mixture for β-elimination that specifically removes the serine- and threonine-phosphate groups from the phospho-peptides. All fractions are then individually re-fractionated by IEF. Peptides sorted to the same fraction as after the first round of IEF have not undergone any modification and are thus tyrosine-phosphorylated or unphosphorylated peptides. However, peptides sorted to a different fraction after re-fractionation have undergone β-elimination and are thus the serine- or threonine-phosphorylated peptides. After separation the latter subsets of phospho-peptides the remaining fractions are subj ected to another round of treatment using phosphatases for enzymatically removing phosphate groups from tyrosine-phosphorylated peptides which are then separated by another round of re-fractionating by IEF. Fig. 2 depicts a schematic illustration of the application of a further preferred embodiment of the invention to the selective enrichment of phospho-peptides. First, proteins are first isotopically labeled using the Isotope-coded Affinity Tag technology (ICAT) and enzymatically digested. Subsequently, the resulting peptides are isobarically labeled using iTRAQ and subjected to the same experimental protocol as described in Fig. 1. Fig. 3 depicts the results of a MALDI-MS and MALDI-MS/MS analysis using double chemical labeled peptides comprised in an immuno-depleted plasma sample. The plasma sample was immuno-depleted by means of a Multiple Affinity Removal LC Column - Human 7 (Agilent Technologies, Inc., Santa Clara, CA, USA). The proteins were isotopically labeled using the ICAT reagent (Applied Biosystems, Inc., Foster City, CA, USA) essentially according to the manufacturer's instructions and digested with trypsin (ratio trypsin:sample = 1:20). Subsequently, the resulting peptides were isobarically labeled essentially following the established iTRAQ protocol (Applied Biosystems, Inc., Foster City, CA, USA). The labeled peptides were processed by strong cation exchange (SCX) fractionation using 50-70-90-100-110-120-130-140-150-180-210-350 mM KCl/ 10mM KH₂PO₄/25% acetonitrile, pH 3.0 and RP-HPLC (20 s fractions, 380 fractions per SCX fraction). MALDI-MS and MALDI-MS/MS analysis were performed on 4579 spots. **Fig.3A** shows a MS analysis of RP-HPLC fraction #12 (SCX fraction 350 mM KCl). Four ICAT peak pairs have been assigned: the expected peak ratio between ICAT(light): ICAT(heavy) = 1.8. **Fig. 3B** shows a MS/MS analysis of ICAT(light) (1655.04 Da) of peak pair #4 (see Fig. 3A). The iTRAQ reporter m/z region (114-117) is enlarged; the expected iTRAQ ratios are: 1:0.1:1:1 **Fig. 3C** shows a MS/MS analysis of ICAT(heavy) (1664.08 Da) of peak pair #4 (see Fig. 3A). The iTRAQ reporter m/z region (114-117) is enlarged; the expected iTRAQ ratios are: 1:1:1:1. **Fig. 3D** shows a MS analysis of RP-HPLC fraction #39 (SCX fraction 50 mM KCL). Four ICAT peak pairs have been assigned; the expected peak ratio between ICAT(light):ICAT(heavy) = 1.5. **Fig. 3E** shows a MS/MS analysis of ICAT(light) (1586.84 Da) of peak pair #4 (see Fig. 3D). The iTRAQ reporter m/z region (114-117) is enlarged; the expected iTRAQ ratios are: 1:0.1:1:1. **Fig. 3F** shows a MS/MS analysis of ICAT(heavy) (1595.87 Da) of peak pair #4 (see Fig. 3D). The iTRAQ reporter m/z region (114-117) is enlarged; the expected iTRAQ ratios are: 1:1:1:1.

### DETAILED DESCRIPTION OF THE INVENTION

The present invention is based on the unexpected finding that combining a particular protein/peptide labeling and fractionation strategy with specific chemical and/or enzymatic reactions targeting the post-translational modification to be analyzed allows the rapid and highly selective enrichment and/or separation of said modified proteins from a complex sample. Furthermore, by adapting the reaction conditions the same method is also appropriate to discriminate between different subsets of proteins bearing a particular post-translational modification.

The present invention illustratively described in the following may suitably be practiced in the absence of any element or elements, limitation or limitations, not specifically disclosed herein.

The present invention will be described with respect to particular embodiments and with reference to certain drawings but the invention is not limited thereto but only by the claims. The drawings described are only schematic and are non-limiting. In the drawings, the size of some of the elements may be exaggerated and not drawn on scale for illustrative purposes.

Where the term "comprising" is used in the present description and claims, it does not exclude other elements or steps. For the purposes of the present invention, the term "consisting of" is considered to be a preferred embodiment of the term "comprising of". If hereinafter a group is defined to comprise at least a certain number of embodiments, this is also to be understood to disclose a group which preferably consists only of these embodiments.

Where an indefinite or definite article is used when referring to a singular noun e.g. "a" or "an", "the", this includes a plural of that noun unless something else is specifically stated.

The term "about" in the context of the present invention denotes an interval of accuracy that the person skilled in the art will understand to still ensure the technical effect of the feature in question. The term typically indicates deviation from the indicated numerical value of ± 10%, and preferably ± 5%.

Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

Further definitions of term will be given in the following in the context of which the terms are used.

In one embodiment, the present invention relates to a method for the selective enrichment and/or separation of phospho-proteins and/or -peptides in a sample, comprising:
(a) single or double chemical labeling of the proteins and/or peptides comprised in the sample
(b) fractionating the proteins and/or peptides;
(c) removing or altering the phosphate-group from at least a first subset of phospho-proteins and/or -peptides;
(d) re-fractionating the proteins and/or peptides;
(e) comparing the fractionation patterns obtained in steps (b) and (d); and
(f) separating the at least first subset of phospho-proteins and/or -peptides modified in step (c) based on the results obtained in step (e).

The term "proteins", as used herein, refers to any naturally occurring or synthetic (e.g., generated by chemical synthesis or recombinant DNA technology) macromolecules comprising a plurality of natural or modified amino acids connected via peptide bonds.

The length of such a molecules may vary from two to several thousand amino acids (the term thus also includes what is generally referred to as oligopeptides). Typically, the term "proteins" relates to molecules having a length of more than 20 amino acids. Thus, proteins to be analyzed in the present invention may have a length from about 30 to about 2500 amino acids, from about 50 to about 1000 amino acids or from about 100 to about 1000 amino acids.

The term "peptide", as used herein, refers to any fragments of the above "proteins" that are obtained after cleavage of one or more peptide bonds. A peptide as used in the present invention is not limited in any way with regard to its size or nature. Typically, peptides to be analyzed in the present invention may have a length from about 2 to about 20 amino acids, from about 3 to about 18 amino acids or from about 5 to about 15 amino acids.

The terms "phospho-proteins" and "phospho-peptides", as used herein, denote any proteins and/or peptides comprising in their primary sequence one or more phosphorylated amino acid residues, particularly phosphorylated serine, threonine or tyrosine residues. Within the scope of the present invention, amino acid phosphorylation may occur *in vivo* by post-translational protein modification or *in vitro* by employing specific protein kinases.

The term "post-translational modification", as used herein, denotes any type of chemical modification of a protein and/or peptide according to the invention that takes place after completion of protein translation. Examples of such modifications include *inter alia* phosphorylation, ubiquitinylation, acetylation, glycosylation, alkylation, isoprenylation, and lipoylation, with phosphorylation being particularly preferred. The term is also to be understood not to be limited with regard to the numbers and/of types of post-translational modifications being comprised in a protein and/or peptide. Thus, a given protein may comprise in its sequence two or more phosphorylated amino acids or one or more phosphorylated amino acids and one or more ubiquitinylated amino acid residues.

The phospho-proteins and/or -peptides are enriched and/or separated by means of the inventive method from a sample comprising such molecules, preferably from a biological sample. The term "sample", as used herein, is not intended to necessarily include or exclude any processing steps prior to the performing of the methods of the invention. The samples can be unprocessed ("crude") samples, extracted protein fractions, purified protein fractions and the like. For example, the samples employed may be pre-processed by immunodepletion of one or more subsets of abundant proteins. Suitable samples include samples of prokaryotic (e.g., bacterial, viral samples) or eukaryotic origin (e.g., fungal, yeast, plant, invertebrate, mammalian and particularly human samples).

The term "complex sample", as used herein, denotes the fact that a sample analyzed using a method of the present invention typically includes a multitude of different proteins and/or peptides (or different variants of such proteins and/or peptides) present in different concentrations. For example, complex samples within the present invention may include at least about 500, at least about 1000, at least about 5000 or at least about 10000 proteins and/or peptides. Typical complex samples used in the invention include *inter alia* cell extracts or lysates of prokaryotic or eukaryotic origin as well as human or non-human body fluids such as whole blood, serum, plasma samples or the like.

The terms "at least a first subset of the phospho-proteins and/or -peptides" and "at least a second subset of the phospho-proteins and/or -peptides", as used herein, are to be understood in such a way that they may relate to the totality of phospho-proteins and/or -peptides present in a given sample or a particular part thereof.

The term "chemical labeling", as used herein, denotes the attachment or incorporation of one or more detectable markers (or "labels") into a protein and/or peptide used in the invention. The term "detectable marker", as used herein, refers to any compound that comprises one or more appropriate chemical substances or enzymes, which directly or indirectly generate a detectable compound or signal in a chemical, physical or enzymatic reaction. As used herein, the term is to be understood to include both the labels as such (i.e. the compound or moiety bound to the protein and/or peptide) as well as the labeling reagent (i.e. the compound or moiety prior to the binding with the peptide or protein). A label used in the present invention may be attached to an amino acid residue of a protein and/or peptide via a covalent or a non-covalent linkage. Typically, the linkage is a covalent linkage. The labels can be selected *inter alia* from isotopic labels, isobaric labels, enzyme labels, colored labels, fluorescent labels, chromogenic labels, luminescent labels, radioactive labels, haptens, biotin, metal complexes, metals, and colloidal gold, with isotonic labels and isobaric labels being particularly preferred. All these types of labels are well established in the art.

The term "single chemical labeling", as used herein, denotes that a protein and/or peptide is labeled with one or more detectable markers of only one type of labels, for example, only isobaric labels. The term "double chemical labeling", as used herein, denotes that a protein and/or peptide is labeled with one or more detectable markers of two different types of labels, for example, isotopic labels and isobaric labels.

In preferred embodiments of the inventive method, the proteins and/or peptides are double labeled. Particularly preferably, the double labeling of the proteins and/or peptides comprises an isotopic labeling and an isobaric labeling, that is the attachment or incorporation of one or more each of isotopic labels and isobaric labels to the proteins and/or peptides to be analyzed, respectively. Within the scope of the present invention, the two labeling steps can be performed in any sequential order or concomitantly. However, in typical embodiments of the inventive method, isotopic labeling precedes the isobaric labeling.

The term "isotopic labeling", as used herein, refers to a labeling event using a set of two or more labels having the same chemical formula but differing from each other in the number and/or type of isotopes present of one or more atoms, resulting in a difference in mass of the proteins and/or peptides labeled that can be detected, for example, via mass spectrometry. In other words, otherwise identical proteins and/or peptides labeled with different isotopic labels can be differentiated as such based on difference in mass. While isobaric labels (see below) in principle constitute a specific type of isotopic labels, in the context of the present invention, the term isotopic label will be used to refer to labels which are not isobaric, but can as such be differentiated based on their molecular weight.

Examples of isotopic labels according to the invention include *inter alia* ¹⁶O-or ¹⁸O-labeled water or Isotope-Coded Affinity Tag (ICAT) labels (cf. Gygi, S.P. et al. (1999) Nat. Biotechnol. 17, 994-999). The ICAT reagent uses three functional elements: a thiol-reactive group for the selective labeling of reduced cysteine amino acid residues, a biotin affinity tag to allow for selective isolation of labeled peptides, and an isotopic tag, which is synthesized in two isotopic forms, the "light" (non-isotopic) and the "heavy" (utilizing, for example, ²H or ¹³C) form. Within the scope of the present invention, isotopic labeling may be performed on the peptide level (e.g., by cleaving the proteins comprised in the sample to be analyzed in the presence of either ¹⁶O- or ¹⁸O-labeled water) or directly on the protein level (i.e. prior to cleavage), for example by employing commercially available ICAT reagents (Applied Biosystems, Foster City, CA, USA).

The term "isobaric labeling", as used herein, refers to a labeling event using a set of two or more labels having the same structure and the same mass, which upon fragmentation release particular fragments having - due to a differential distribution of isotopes within the isobaric labels - the same structure but differ in mass. Isobaric labels typically comprise a reporter group which in mass spectrometric analyses generates a strong signature ion upon collision induced dissociation (CID, i.e. a fragment release) and a balance group which comprises a certain compensating number of isotopes so as to ensure that the combined mass of the reporter group and balance group is constant for the different isobaric labels. The balance group may or may not be released from the label upon CID.

Examples of isobaric labels according to the invention include *inter alia* Isobaric Tag for Relative and Absolute Quantitation (iTRAQ) labels (cf. Ross, P.L. et al. (2004) Mol. Cell. Proteomics 3, 1154-1169). This approach employs four different iTRAQ reagents, each containing a reporter group, a balance group and a peptide reactive group which reacts with primary amine groups (for example, the ε amino-group of lysine amino acid residues). The reporter group has a mass of 114, 115, 116 or 117 Da, depending on differential isotopic combinations of ¹²C/¹³C and ¹⁶O/¹⁸O in each reagent. The balance group varies in mass from 31 to 28 Da to ensure that the combined mass of the reporter group and the balance group remains constant (145 Da) for the four reagents. Accordingly, labeling of the same peptide with each of these reagents results in peptides which are isobaric and thus co-elute, for example, in liquid chromatography and consequently are chromatographically indistinguishable from each other. During mass spectrometry, however, at least the respective reporter groups are released upon CID, displaying distinct masses of 114 to 117 Da. The intensity of these fragments can be used for quantification of the individual proteins and/or peptides in a single.

In preferred embodiments of the invention, the method further comprises single or double labeling of the proteins and/or peptides comprised in the samples prior to subjecting them to fractionation.

The present invention particularly relates to the combined use of isotopic labels and isobaric labels for multiplexed protein analysis, that is for performing multiple analyses in parallel, for example 2, 4, 8 or 16 parallel samples. In particular, such a combined labeling strategy also enables the comparison of relative protein levels between different samples. The combination of isotopic and isobaric labeling may have the advantage that only those peptides need to be specifically analyzed, for example by MALDI-MS/MS analysis or iTRAQ quantification, for which a differential expression level is observed, thus resulting in a faster and less complex sample analysis.

The terms "fractionation" and "re-fractionaction", as used herein, refer to any type separation process in which the proteins and/or peptides present in a sample in a certain quantity are divided (i.e. sorted) up in a large number of smaller quantities (i.e. fractions) according to any differences in their physico-chemical properties such as the molecular mass, their size, and their overall net charge. A common trait in fractionations is the need to find an optimum between the amount of fractions collected and the desired purity in each fraction. Fractionation makes it possible to isolate more than two components in a mixture in a single run. This property sets it apart from other separation techniques. There are several methods for fractionating proteins and/or peptides well established in the art including classical SDS polyacrylamide gel electrophoresis (SDS-PAGE), two-dimensional gel electrophoresis, size-exclusion chromatography, (two-dimensional) liquid chromatography, and isoelectric focusing, with the latter one being particularly preferred. Methods for analysis, particularly visualization, of the proteinaceous molecules after fractionation has taken place are well established in the art.

Isoelectric focusing is a technique for separating different molecules by their electric charge differences. It is a type of zone electrophoresis, usually performed in a gel (e.g., an agarose gel or, preferably, polyacrylamide gel) or in liquid phase, that takes advantage of the fact that a molecule's charge changes with the pH of its surroundings. The molecules to be focused are distributed over a medium that has a pH gradient. An electric current is passed through the medium, creating a "positive" anode and "negative" cathode end. Negatively charged molecules migrate through the pH gradient in the medium toward the "positive" end while positively charged molecules move toward the "negative" end. As a particle moves towards the pole opposite of its charge it moves through the changing pH gradient until it reaches a point in which the pH of that molecules isoelectric point (pI) is reached. At this point the molecule no longer has a net electric charge (due to the protonation or deprotonation of the associated functional groups) and as such will not proceed any further within the gel. Isoelectric focusing can resolve proteins that differ in pI value by as little as 0.01.

Isoelectric focusing is usually the first step in two-dimensional gel electrophoresis, in which proteins are first separated by their pI and then further separated by molecular weight through standard SDS-PAGE.

The term "re-fractionating", as used herein, also denotes that the method of the invention may be performed with a single type of fractionation method, that is, the proteins and/or peptides present in a sample are fractionated before and after removing and/or altering a specific post-translational modification, particularly a phosphate group, by applying the same method, preferably isoelectric focusing. However, depending on the particular protocol it may also be possible to use different methods, e.g. isoelectric focusing and classical SDS-PAGE.

Other methods of fractionation/re-fractionation and combinations thereof may also be envisaged. Thus, fractionation/re-fractionation may rely on ion exchange chromatography, size exclusion chromatography, hydrophobic interaction chromatography, reversed-phase chromatography and/or (immuno)affinity chromatography.

In preferred embodiments, the method further comprises cleaving the proteins into peptides prior to subjecting them to fractionation. Such cleaving of proteins may either be achieved chemically (e.g., via acid or base treatment employing chemicals such as cyanogen bromide, 2-(2'-nitrophenylsulfonyl)-3-methyl-3-bromo-indolenine (BNPS), formic acid, hydroxylamine, iodobenzoic acid, and 2-nitro-5-thiocyanobenzoid acid) or enzymatically via proteases (including *inter alia* trypsin, pepsin, thrombin, papain, and proteinase K) well known in the art.

In particular, the present invention relates to the selective enrichment and/or separation of phospho-proteins and/or -peptides. In other words, the post-translational modification of the at least first subset of the proteins and/or peptides to be analyzed that is to be removed or altered is a phosphate-group. The term "removing", as used herein, refers to the complete elimination of the phosphate-group, for example by chemical cleavage or enzyme action (see also the discussion below). The term "altering", as used herein, denotes any modification of the phosphate-group resulting in a change in the physico-chemical properties of the proteins and/or peptides that allows a discrimination between the variants bearing the original and the altered post-translational phosphate-group. Examples of such alterations include *inter alia* the cleavage of one or more chemical bonds within the phosphate group without removing it from the amino acid backbone as well as the conjugation of the phosphate group with any moiety, thus resulting in an increase in molecular weight. Typically, the treatment of the proteins and/or peptides is performed under reaction conditions ensuring the removal and/or alteration of all post-translational phosphate-groups that are comprised in the molecules in order to avoid the occurrence of any "intermediate molecules" where one or more phosphate-groups have been removed and/or altered but one or more others remain unchanged.

The removal and/or alteration of the post-translational phosphate modification results in an alteration of the physico-chemical properties of only the at least first subset of phospho-proteins and/or -peptides compared to the remaining proteins and/or peptides present in the sample (optionally including any at least second subset of phospho-proteins and/or -peptides) such that this at least first subset can be identified during the subsequent re-fractionation step, since only this at least first subset will sort into a different fraction. All other proteins and/or peptides comprised in the sample will sort into the same fraction as during the initial fractionation step prior to removing/altering the phosphate-group from the at least first subset of phospho-proteins and/or -peptides.

Thus, for identifying and/or separating the at least first subset of phospho-proteins and/or -peptides the results obtained in the initial and the re-fractionation step have to be compared (for example, comparing the specific patterns obtained after staining the gel used for electrophoretic protein separation).

In order to facilitate the sorting/separation process the fractions obtained after the initial fractionation step are preferably treated individually to removing/altering the post-translational phosphate-group before applying them to re-fractionation.

In further preferred embodiments of the invention, the method is used for the discrimination of different subsets of phospho-proteins and/or -peptides, namely serine/threonine-phosphorylated (i.e. the phosphate is attached to a serine or a threonine amino acid residue) and tyrosine-phosphorylated proteins and/or peptides (i.e. the phosphate is attached to a tyrosine amino acid residue). Since these subsets of phospho-proteins and/or -peptides are regulated by different kinases and phosphatases, they have also been implicated in different cellular processes or signaling cascades.

Importantly, the two above subsets of phospho-proteins and/or -peptides also differ in their accessibility to chemical treatment with regard to the removal and/or alteration of post-translational modifications. For example, serine- and threonine-phosphorylated proteinaceous molecules are accessible to the chemical removal of the phosphate group via β-elimination (i.e., a type of elimination reaction well established in the art, wherein atoms or atom groups are removed from two adjacent atoms of the substrate while forming a π bond), whereas tryrosine-phosphorylated proteinaceous molecules are not. This difference provides a basis for distinguishing these to subsets of phospho-proteins and/or -peptides.

Thus, in preferred embodiments of the invention, the method is configured to allow the discrimination between serine- and threonine-phosphorylated and tyrosine-phosphorylated proteins and/or peptides, respectively. Based on the above-mentioned accessibility to chemical treatment it is particularly preferred that the at least first subset of phospho-proteins and/or -peptides comprises serine- and threonine-phosphorylated proteins and/or peptides. In other words, following the initial fractionation step the first subset to be enriched and/or separated from tyrosine-phosphorylated and unphosphorylated proteins and/or peptides comprises serine- and threonine-phosphorylated proteins and/or peptides.

Preferably, in any embodiments of the invention relating to the analysis of phospho-proteins and/or -peptides the phosphate-group is removed chemically via β-elimination. However, it is also possible to remove the phosphate-group, for example, enzymatically by means of specific or non-specific phosphatases.

To specifically remove the phosphate moieties from phospho-serine and phospho-threonine amino acid residues, β-elimination is typically induced by base hydrolysis. In order to avoid any adverse effects on the side chains of cysteine and methionine residues, the samples may first be treated with performic acid, resulting in the oxidation, and thus inactivation of these residues. A typical reaction mixture for performing β-elimination comprises H₂O, dimethyl sulfoxide, acetonitrile, 250 mM EDTA (pH 8.0), and 5 M NaOH.. The samples are incubated for 1 h at 55°C under a N₂ atmosphere, cooled to room temperature, and quenched by neutralizing with acetic acid. Removing the phosphate-group via β-elimination results in forming an unnatural amino acid (e.g., dehydro-alanine, dehydro-2-amino butyric acid).

After subjecting the individual fractions obtained after the initial fractionation step to the above treatment, the are re-fractionated, typically by applying the same fractionation method, preferably isoelectric focusing.

The removal and/or alteration of the phosphate group specifically from phospho-serine and phospho-threonine amino acid residues results in an alteration of the physico-chemical properties of only those subset of proteinaceous molecules (i.e. the at least first subset) comprising such modified amino acid residues such that they can be identified during the subsequent re-fractionation step, since only this subset will sort into a different fraction. All other proteins and/or peptides comprised in the sample including tyrosine-phosphorylated and - of course - unphosphorylated proteins and/or peptides will sort into the same fraction as during the initial fractionation step prior to removing/altering the post-translational phosphate-group. Thus, using the above-described method serine- and threonine-phosphorylated proteins and/or peptides can be selectively enriched from a complex sample by comparing the results of the initial and the re-fractionation step performed.

In another embodiment, the inventive method further comprises the selective enrichment and/or separation of at least a second subset of phospho-proteins and/or -peptides from the subset of proteins and/or peptides remaining after separation of the at least first subset following the re-fractionation step, as described above. To accomplish this goal, the remaining subset of proteins and/or peptides is subjected to another cycle of fractionation, removing or altering the post-translational phosphate modification, and re-fractionation, wherein the phosphate-group from at least a second subset of phospho-proteins and/or -peptides is removed or altered. Particularly preferably, the at least second subset of proteinaceous molecules is tyrosine-phosphorylated proteinaceous molecules. Since phosphorylated tyrosine residues are not accessible to chemical cleavage via β-elimination, it is preferred to remove the phosphate group enzymatically. Enzymatic removal may be achieved via phosphatases, particularly via alkaline phosphatases (for the non-specific removal of phosphate-groups) or via protein tyrosine phosphatases (for specifically dephosphorylating phospho-tyrosine residues). Such phosphatases are commercially available.

By performing a second re-fractionation step the tyrosine-phosphorylated proteins and/or peptides can be separated from their unphosphorylated counterparts based on the same principle as described above. The remaining subset of proteins and/or peptides may optionally be subjected to a third, forth, and so forth cycle of fractionation, removing/altering a post-translational modification, and re-fractionation, wherein the type of post-translational modification to be removed or altered is typically not phosphorylation but, for example, ubiquitinylation or glycosylation.

In other embodiments, the method further comprises analyzing the separated phospho-proteins and/or -peptides by means of mass spectrometry, an analytical technique used to measure the mass-to-charge ratio of ions. Importantly, for phospho-proteins and/or -peptides mass spectrometric analysis is facilitated by the removal of the unstable phosphate-group but leaving behind a unique unnatural amino acid for the serine and threonine-phosphorylated species. The particular mass spectrometric analysis applied may depend on the levels of protein and/or peptide expression determined in different samples. In some embodiments, the method of the invention are performed in a high-throughput format.

In a further embodiment, the invention relates to the use of a method, as described herein, for performing qualitative and/or quantitative proteomic analyses.

While the above invention has been described with respect to some of its preferred embodiments, this is in no way to limit the scope of the invention. The person skilled in the art is clearly aware of further embodiments and alterations to the previously described embodiments that are still within the scope of the present invention.

### EXAMPLES

### Example 1

Commercially available instrumentation such as the IPGPhor IEF unit (Amersham-Pharmacia, Piscataway, NJ, USA) was used for electrophoretic separation of the protein and/or peptide samples employed according to their isoelectric point. Chemical or enzymatic digestion of the proteins into peptides was optionally performed following established standard protocols.

To specifically remove the phosphate moieties from phosphoserine and phosphothreonine amino acid residues, base hydrolysis was used to induce β-elimination. In order to avoid any adverse effects on the side chains of cysteine and methionine residues, the samples were first treated with performic acid, resulting in the oxidation of these residues, thereby inactivating them (Goshe, M.B. et al.(2001) Anal. Chem. 73, 2578-2586).

The β-elimination reaction mixture used was as follows: H₂O, dimethyl sulfoxide, acetonitrile, 250 mM EDTA (pH 8.0), and 5 M NaOH. All solvents were degassed with N₂ before and after preparation of the reaction mixture.

The β-elimination reaction mixture was added to the lyophilized protein fractions isolated from the gel after isoelectric focussing. The samples were incubated for 1 h at 55°C under a N₂ atmosphere, cooled to room temperature, and the reaction quenched by neutralizing with acetic acid (Goshe, M.B. et al.(2001), *supra*)

Commercially available phosphates were to specifically dephosphorylate phospho-tyrosine amino acid residues.

By treating the protein and/or peptide samples in the above-described manner and subsequent electrophoretic separation of the respective fractions according to their isoelectric point serine/threonine-phosphorylated proteins and/or peptides as well as tyrosine-phosphorylated proteins and/or peptides could be selectively enriched and separated.

### Example 2

Initially, a human blood plasma sample was immuno-depleted using a Multiple Affinity Removal LC Column - Human 7 (4.6 x 10 mm; Agilent Technologies, Inc., Santa Clara, CA, USA) according to the manufacturer's instructions. Seven highly abundant plasma proteins - albumin, IgG, antitrypsin, IgA, transferrin, fibrinogen, and haptoglobin - that constitute approximately 85% of the total protein mass of human plasma were removed by this treatment. The column requires a two buffer system for operation. Buffers A and B are optimized to minimize co-adsorption of non-targeted proteins and to ensure reproducibility of column performance and long column lifetime. Buffer A - a salt-containing neutral buffer, pH-7.4 - was used for loading, washing, and re-equilibrating the column, and Buffer B - a low pH urea buffer - was used for eluting the bound high-abundant proteins from the column. The depletion was performed using an Agilent 1200 series LC system. Before injection onto the column serum from human male AB plasma, sterile-filtered (Sigma- H4522) was diluted 4X with Buffer A. The sample was transferred to a 0.22 µm spin tube and centrifuged for 1 min. at 16,000 x g to remove particulates. Diluted plasma was kept at 4°C.

The proteins were isotopically labeled using the ICAT reagent (Applied Biosystems, Inc., Foster City, CA, USA) essentially according to the manufacturer's instructions and digested with trypsin (ratio trypsin:sample = 1:20). Subsequently, the resulting peptides were isobarically labeled essentially following the established iTRAQ protocol (Applied Biosystems, Inc., Foster City, CA, USA).

However, the buffers used for ICAT are incompatible with iTRAQ labeling and *vice versa*. Since labeling and purifying the samples consecutively is time-consuming and does not take full advantage of the multiplexing procedure, the buffer composition was optimized in such way that one buffer composition can be used for both labeling reactions. Furthermore, Agilent buffer A is incompatible with iTRAQ labeling as well. Therefore, the flow-through fractions were subjected to a buffer-exchange procedure using 5K MWCO spin concentrators (Agilent Technologies) to ensure using an appropriate buffer compatible with both ICAT and iTRAQ labeling chemistries. 50 mM triethyl ammonium bicarbonate (TEAB) was found to be optimal.

The order of labeling is also important, since first performing an iTRAQ labeling without blocking cysteine amino acid residues (which are used for the ICAT labeling), results in a significantly reduced efficiency of protein digestion. The amount of starting material was 50 µg protein (as determined via a Bradford assay; Bio-Rad Laboratories, Hercules, CA, USA) dissolved in 40 µl 50 mM triethyl ammoniumbicarbonate (TEAB) (for ICAT labeling, the concentration should be 1.2 mg/ml). To each sample, 1 µl 50 mM TCEP (Tris(2-carboxyethyl) phosphine hydrochloride) and 2µl 2% SDS were added and the mixtures were boiled for 10 minutes. Then, 20 µl acetonitrile were added to each sample. The samples were allowed to cool and each added to a vial of ICAT reagent. Incubation was done for 2 hours at 37°C.

For cleavage of the proteins, trypsin was added at a 1:20 ratio and the samples were incubate for 12-16 hours. Then, the samples were completely dried in a speed-vac. 30 µl 1M TEAB and 70 µl acetonitrile were pre-mixed per iTRAQ label and added to each ICAT labeled protein digest. Re-solubilization of the peptides should be ensured. This mixture was added to the iTRAQ reagent tube and labeled for 1 hour at room temperature. To quench the reaction, 100 µl MilliQ-purified water was added to each tube and the tubes were incubated for 30 min. at room temperature. Finally samples were dried using a centrifugal vacuum concentrator

For sample purification, to each of the sample tubes 250 µl CEX loading buffer (10 mM KH₂PO₄/25% acetonitrile (ACN), pH 3.0) were added. Samples were vortexed and sonicated during three cycles to maximize re-solubilisation of the peptides. The samples were combined and diluted to a total volume of 4 ml CEX loading buffer. Perform SCX and avidin sample clean up according to manufacturer's protocol and cleave the ICAT according to the following protocol:
- Check the pH using pH paper. If the pH is not between 2.5 and 3.3, adjust by adding more Cation Exchange Buffer-Load.
- To condition the CEX cartridge, inject 2 ml of the Cation Exchange Buffer-Load. Divert to waste.
- Slowly inject (∼1 drop/second) the diluted sample mixture onto the cation-exchange cartridge and collect the flow-through into a sample tube.
- Inject 1 ml of the Cation Exchange Buffer-Load to wash the TCEP, SDS, and excess ICAT reagents from the cartridge.
- To elute the peptides, slowly inject (∼1 drop/second) 500 µl of the Cation Exchange Buffer-Elute (10mM KH₂PO₄/ 500mM KCl / 25% ACN, pH 3.0). Capture the eluate in a fresh 1.5-mL tube. Collect the eluted peptides as a single fraction.

The purification of the biotinylated peptides and the removal of the biotin label were performed as follows:
- Inject 2 ml of the Affinity Buffer-Elute (30% ACN / 0.4% TFA) onto the avidin cartridge. Divert to waste.
- Inject 2 ml of the Affinity Buffer-Load (2 x PBS, pH 7.2). Divert to waste.
- Neutralize each cation-exchange fraction by adding 1 ml of the Affinity Buffer-Load.
- Check the pH using pH paper. If the pH is not 7, adjust by adding more Affinity Buffer-Load.
- Vortex to mix, then centrifuge for a few seconds to bring all solution to the bottom of the tube.
- Label three fraction-collection tubes: #1 (Flow-Through), #2 (Wash), and #3 (Elute), then place in a rack.
- Slowly inject (∼1 drop/5 seconds) of the neutralized fraction onto the avidin cartridge and collect the flow-through into tube #1 (Flow-Through).
- Inject 500 µl of Affinity Buffer-Load onto the cartridge and continue to collect in tube #1 (Flow-Through)
- To reduce the salt concentration, inject 1 ml of Affinity Buffer- Wash 1 (PBS, pH 7.2). Divert the output to waste.
- To remove nonspecifically bound peptides, inject 1 ml of Affinity Buffer-Wash 2 (50mM NH₄HCO₃/20% MeOH, pH 8.3). Collect the first 500 µl in tube #2 (Wash). Divert the remaining 500 µl to waste.
- Inject 1 ml of MilliQ-purified water or equivalent. Divert to waste.
- Fill a syringe with 800 µl of the Affinity Buffer-Elute.
- To elute the labeled peptides, slowly inject (∼1 drop/5 seconds) 50 µl of the Affinity Buffer-Elute and discard the eluate.
- Inject the remaining 750 µl of Affinity Buffer-Elute and collect the eluate in tube #3 (Elute).
- Vortex to mix, then centrifuge for a few seconds to bring all solution to the bottom of the tube.
- Evaporate each affinity-eluted fraction to dryness in a centrifugal vacuum concentrator.
- In a fresh tube, prepare the final cleaving reagent by combining TFA (Cleaving Reagent A) and Cleaving Reagent B (Applied Biosystems) in a 95:5 ratio. - 90 µl of final cleaving reagent per sample are required.
- Vortex to mix, then centrifuge for a few seconds to bring all solution to the bottom of the tube.
- Transfer -90 µl of freshly prepared cleaving reagent to each sample tube.
- Vortex to mix, then centrifuge for a few seconds to bring all solution to the bottom of the tube.
- Incubate for 2 hours at 37 °C.
- Centrifuge the tube for a few seconds to bring all solution to the bottom of the tube.
- Evaporate the sample to dryness in a centrifugal vacuum concentrator (∼30 to 60 min).

Multidimensional liquid chromatography (SCX-RP-LC), or alternatively isoelectric focusing of peptides followed by reverse-phase liquid chromatography (RF-LC), or 1D (RP)-LC was used to separate peptides. MALDI-MS and MALDI-MS/MS analysis were performed on 4579 spots using a 4800 MALDI-ToF-ToF analyzer (Applied Biosystems, Inc.).

**Fig.3A** shows a MS analysis of RP-HPLC fraction #12 (SCX fraction 350 mM KCl). Four ICAT peak pairs have been assigned: the expected peak ratio between ICAT(light): ICAT(heavy) = 1.8. **Fig. 3B** shows a MS/MS analysis of ICAT(light) (1655.04 Da) of peak pair #4 (see Fig. 3A). The iTRAQ reporter m/z region (114-117) is enlarged; the expected iTRAQ ratios are: 1:0.1:1:1 **Fig. 3C** shows a MS/MS analysis of ICAT(heavy) (1664.08 Da) of peak pair #4 (see Fig. 3A). The iTRAQ reporter m/z region (114-117) is enlarged; the expected iTRAQ ratios are: 1:1:1:1. **Fig. 3D** shows a MS analysis of RP-HPLC fraction #39 (SCX fraction 50 mM KCL). Four ICAT peak pairs have been assigned; the expected peak ratio between ICAT(light):ICAT(heavy) = 1.5. **Fig. 3E** shows a MS/MS analysis of ICAT(light) (1586.84 Da) of peak pair #4 (see Fig. 3D). The iTRAQ reporter m/z region (114-117) is enlarged; the expected iTRAQ ratios are: 1:0.1:1:1. **Fig. 3F** shows a MS/MS analysis of ICAT(heavy) (1595.87 Da) of peak pair #4 (see Fig. 3D). The iTRAQ reporter m/z region (114-117) is enlarged; the expected iTRAQ ratios are: 1:1:1:1.

## Claims

1. Method for the selective enrichment and/or separation of phospho-proteins and/or -peptides in a sample, comprising:
(a) single or double chemical labeling of the proteins and/or peptides comprised in the sample
(b) fractionating the proteins and/or peptides;
(c) removing or altering the phosphate-group from at least a first subset of phospho-proteins and/or -peptides;
(d) re-fractionating the proteins and/or peptides;
(e) comparing the fractionation patterns obtained in steps (b) and (d); and
(f) separating the at least first subset of phospho-proteins and/or -peptides modified in step (c) based on the results obtained in step (e).

2. The method of claim 1, wherein the fractionation/re-fractionation is performed via isoelectric focusing.

3. The method of claim 1 or 2, further comprising: cleaving the proteins into peptides prior to subjecting them to fractionation.

4. The method of any of claims 1 to 3, further comprising: single or double chemical labeling of the proteins and/or peptides comprised in the sample prior to subjecting them to fractionation.

5. The method of claim 4, wherein the double labeling comprises an isotopic and an isobaric labeling.

6. The method of claim 5, wherein the isotopic labeling is performed prior to cleaving the proteins and/or peptides.

7. The method of any of claims 1 to 6, wherein the at least first subset of phospho-proteins and/or -peptides comprises serine- and threonine-phosphorylated phospho-proteins and/or -peptides.

8. The method of claim 7, wherein the phosphate-group is removed chemically via β-elimination.

9. The method of any of claims 1 to 8, wherein after performing step (e) the remaining subset of proteins and/or peptides is subjected to another cycle of steps (a) to (e), and wherein step (b) comprises removing or altering a specific post-translational modification from at least a second subset of phospho-proteins and/or -peptides.

10. The method of claim 9, wherein the at least second subset of phospho-proteins/peptides comprises tyrosine-phosphorylated phospho-proteins and/or -peptides.

11. The method of claim 10, wherein the phosphate-group is removed enzymatically via phosphatases.

12. The method of any of claims 1 to 11, further comprising: analyzing the separated phospho-proteins and/or -peptides by means of mass spectrometry.

13. The method of any of claims 1 to 12, wherein the method is performed in a high-throughput format.

14. Use of a method of any of claims 1 to 13 for performing qualitative and/or quantitative proteomic analyses.
